# EUROPEAN PATENT SPECIFICATION

(11) **EP 4 010 037 B1**
(45) Date of publication and mention of the grant of the patent: **25.02.2026**
(21) Application number: 20853237.4
(22) Date of filing: 10.08.2020
(51) Int. Cl.: A61F 13/0206, A61F 13/0246, A61L 15/58, C09J 7/29, C09J 7/38

(54) **LOW TRAUMA WOUND DRESSINGS AND ADHESIVES FOR SAME**
VERBÄNDE FÜR LEICHTE WUNDEN UND KLEBEMITTEL DAFÜR
PANSEMENTS À FAIBLE TRAUMATISME ET ADHÉSIFS POUR CEUX-CI

(30) Priority: 09.08.2019 US 201962884700 P
(43) Date of publication of application: 15.06.2022
(73) Proprietor: Mativ Luxembourg, 5326 Contern (LU)
(72) Inventor: EGAN, Michael, Kiernan, Swanland Yorkshire HU14 3QS (GB); YATES, Michael, Stuart, Mirfield Yorkshire WF14 9TP (GB)
(74) Representative: Richardt Patentanwälte PartG mbB
(86) International application number: PCT/US2020/045606
(87) International publication number: WO 2021/030259

(56) References cited:
- EP-B1- 0 597 636
- EP-B1- 2 436 380
- US-A1- 2002 160 037
- US-A1- 2009 216 168
- US-A1- 2017 051 189
- US-B1- 6 191 216
- US-B2- 7 759 537
- ANONYMOUS .: "POLYETHYLENE FILM ", PIEDMONT PLASTICS, pages 1 - 3, XP055900038, Retrieved from the Internet <URL:https://web.archive.org/web/20201113143219/https://www.piedmontplastics.com/products/polyethylene-film> [retrieved on 20220311]

## Description

### CROSS-REFERENCE TO RELATED APPLICATIONS

This application claims the benefit of U.S. Provisional Application Serial No. 62/884,700, filed August 9, 2019.

### BACKGROUND

The present disclosure relates to adhesives and sealable wound dressings such as bandages having adhesives, and more particularly to sealable wound dressings having an easy peel-off adhesive for low trauma release, repositioning and/or resealing of the wound dressing.

Wound dressings such as bandages are commonly used in medical applications to protectively cover, bind up, apply pressure to, and/or facilitate healing of wounds such as cuts, scrapes, scratches, scabs, punctures, and minor burns and scars on the skin. These bandages may comprise a strip of flexible cloth or other material that can cover, strengthen, or compress the wound. Some bandages also include a gauze or absorbent pad that can absorb fluids such as blood, sweat, inflammatory fluids, exudate, or other discharge near the wound, while also serving to cushion the wound. These pads may further contain or be used to apply medicinal or biological agents to help the would heal, such as ointments that have antimicrobial effects. Other bandages may simply provide compression when wrapped around the wound.

One particularly desirable aspect for these types of bandages is a strong seal strength or bond, and more particularly, its ability to remain secured to the patient, or to itself, if the bandage is of the type to be wrapped around the wound to apply compression. The ability of an adhesive to stick immediately is referred to as "tack." Tack is important in wound care dressings and other bandages because it is necessary to hold the dressing in the correct place. In an ideal scenario, there would be sufficient tack to secure the dressing in place during application, adhesion would keep the dressing in place for the duration of the appropriate wear time.

At the same time, the dressing should also permit removal without causing skin trauma, like tears or stripping. Certain types of wounds require frequent redressing with a fresh, clean bandage, while in other situations the duration of the application of the dressing is relatively short. Sometimes, the bandage is inadvertently positioned incorrectly. The ability to reposition the bandage without sacrificing its integrity is beneficial.

In addition, skin tears and stripping are always considerations when dealing with older and younger patients. The result of shearing, friction, or blunt trauma, skin tears result in the separation of the layers of skin. Skin tears are painful and can lead to infections and enlargement of the original wound. Thus, it is desirable to provide a bandage having adequate adhesion levels during device attachment and low mechanical trauma during removal.

EP0597636 and US2002/160037 disclose prior art bandages.

Unfortunately, with traditional bandages the greater the seal strength, the harder the releasability. In most cases, difficulty removing the bandage is equally as undesirable as weakness of the seal strength. In some bandages, the difficulty of release also results in pain and discomfort to the patient during removal, particularly when the bandage is applied to sensitive areas of the patient's skin, such as on regions having hair or existing cuts, lesions or other skin defects.

Accordingly, it would be desirable to provide a bandage having a seal strength that is sufficient to allow it to be used in medical applications, and a low trauma, peel-off releasability that does not cause pain or discomfort to the patient, and a low trauma, peel-off releasable and resealable adhesive suitable for use in such a bandage, or any wound dressing or wearable medical device.

### SUMMARY

The present disclosure provides a wound dressing such as a bandage having a seal strength that is sufficient to allow it to be used in medical applications, and a low trauma, peel-off releasability for the convenience and comfort of the patient. A low trauma, peel-off releasable and resealable adhesive for use with such a bandage, or any wound dressing or wearable medical device, is also provided.

In one aspect of the invention, a wound dressing in the form of a bandage may be provided. The wound dressing comprises a film base having a lower surface for contact with skin, an opposed, upper surface, and a polyurethane adhesive layer extending across at least a portion of the lower surface of the film base. The polyurethane adhesive includes a mixture of: (a) a polyol mixture, (b) an isocyanate, and (c) a catalyst mix.

The adhesive layer has a thickness in the range of about 73 to about 384 µm and an adhesion value of less than about 150 g/cm when tested against a metal plate. In some embodiments, the thickness of the adhesive is in the range of about 50 to about 80 µm, and the adhesion value is less than about 100 g/cm against a metal plate. Accordingly, the adhesive of the present disclosure can allow for easy atraumatic release of the wound dressing, while still having a seal strength that is sufficient to allow it to be used in medical applications. In addition, the adhesive of the present disclosure allows the bandage to be repositioned without sacrificing its integrity (i.e., without loss of adhesion or deformation of bandage structure) and without causing trauma, discomfort or pain to the patient.

The wound dressing further includes an absorbent pad component attached to the lower surface of the film base. The adhesive layer may be provided as a coating, and the adhesive may be a pressure-sensitive adhesive. In certain embodiments, the adhesive layer may include a flexible substrate film for carrying the adhesive. The substrate film may be selected from the group consisting of polyethylene, polyethylene terephthalate, polypropylene, polyurethane, ethylene vinyl acetate (EVA), polyester, copolyesters, and other polymer blends. The substrate film may be a fabric, woven, non-woven, or sheet material.

In another embodiment, an adhesive is provided. The adhesive comprises a polyurethane adhesive including a mixture of: (a) a polyol mixture, (b) an isocyanate, and (c) a catalyst mix. The adhesive has a thickness in the range of about 73 to about 384 µm and an adhesion value of less than about 150 g/cm against a metal plate. In some embodiments, the thickness of the adhesive is about 50 to about 80 µm and the adhesion value is less than about 100 g/cm against a metal plate. The adhesive may be a pressure-sensitive adhesive.

The adhesive may be a coating on a medical device, such as a wound dressing. The wound dressing may be a bandage, in some embodiments. The adhesive may also be carried on a substrate film. The substrate film may be selected from the group consisting of polyethylene, polyethylene terephthalate, polypropylene, polyurethane, ethylene vinyl acetate (EVA), polyester, copolyesters, and other polymer blends. In some embodiments, the substrate film may be a fabric, woven, non-woven, or sheet material, and may be flexible.

It is to be understood that both the foregoing general description and the following detailed description are exemplary and explanatory only and are not restrictive of the disclosure. Additional features of the disclosure will be set forth in part in the description which follows or may be learned by practice of the disclosure.

### BRIEF DESCRIPTION OF THE DRAWINGS

The accompanying drawings, which are incorporated in and constitute a part of this specification, illustrate several embodiments of the disclosure and together with the description, serve to explain the principles of the disclosure.
FIG. 1 illustrates a schematic diagram of an underside view of an exemplary bandage in accordance with an embodiment of the present disclosure.
FIG. 2 illustrates a schematic diagram of a cross-sectional view of the bandage of FIG. 1.
FIG. 3 is a graph showing results from an adhesion test using a metal plate and two exemplary embodiments of an adhesive carried on substrate films (EU38 and EU50) of the present disclosure.
FIG. 4 is a graph showing the results of varying the catalyst content, isocyanate content, oven temperature and time in the oven for the EU38 film of FIG. 3.
FIG. 5 is a bar graph showing adhesion strength to a metal plate of an exemplary embodiment of an adhesive of the present disclosure in comparison with other adhesives.
FIG. 6 is a bar graph showing adhesion strength to a Stratex^{®} pad of the adhesive of FIG. 5 in comparison with other adhesives.
FIG. 7 is a bar graph showing adhesion strength to an embossed polyethylene (PE) film of the adhesive of FIG. 5 in comparison with other adhesives.
FIG. 8 is a bar graph showing combined results from FIGS. 5, 6 and 7.
FIG. 9 is a photograph showing a side-by-side comparison of the adhesive of the present disclosure and another adhesive, both after 30 minutes total immersion in water.
FIG. 10 is a photograph showing a side-by-side comparison of the adhesive of the present disclosure on two different film substrates, after 1 minute of total immersion in water.
FIG. 11 is a photograph of the adhesive of the present disclosure on an EU38 film substrate.

### DESCRIPTION OF THE EMBODIMENTS

This description and the accompanying drawings illustrate exemplary embodiments and should not be taken as limiting, with the claims defining the scope of the present disclosure. Various mechanical, compositional, structural, and operational changes may be made without departing from the scope of this description and the claims. In some instances, well-known structures and techniques have not been shown or described in detail so as not to obscure the disclosure. Like numbers in two or more figures represent the same or similar elements. Furthermore, elements and their associated aspects that are described in detail with reference to one embodiment may, whenever practical, be included in other embodiments in which they are not specifically shown or described. For example, if an element is described in detail with reference to one embodiment and is not described with reference to a second embodiment, the element may nevertheless be claimed as included in the second embodiment. Moreover, the depictions herein are for illustrative purposes only and do not necessarily reflect the actual shape, size, or dimensions of the system or illustrated components.

It is noted that, as used in this specification and the appended claims, the singular forms "a," "an," and "the," and any singular use of any word, include plural referents unless expressly and unequivocally limited to one referent. As used herein, the term "include" and its grammatical variants are intended to be non-limiting, such that recitation of items in a list is not to the exclusion of other like items that can be substituted or added to the listed items.

For purposes of illustration, the embodiments are described with reference to medical applications. However, the embodiments may be implemented in any application requiring adhesion and removal to a surface. Further, it is understood that the illustrations are not drawn to scale and therefore is not intended to limit the invention in any way.

The present disclosure provides an adhesive that can be used with a variety of wound dressings to provide low trauma, peel-off releasability and resealability, while still having a seal strength that is sufficient to allow it to be used in medical applications. In one exemplary embodiment, the wound dressing may take the form of a bandage.

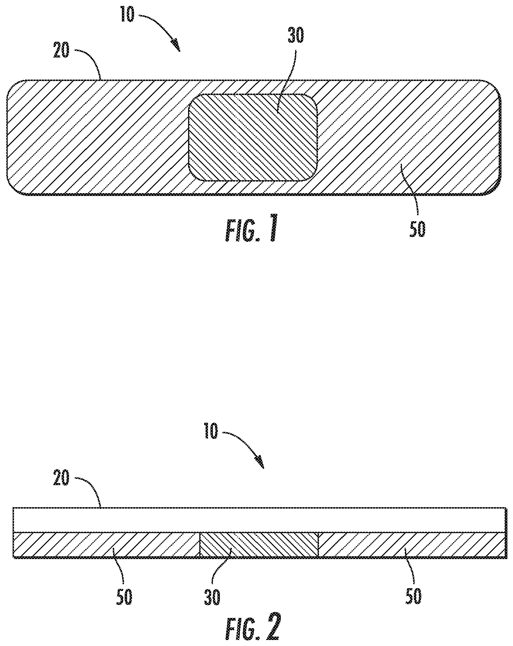

FIG. 1 shows a schematic diagram of the lower surface or underside of an exemplary wound dressing in the form of a bandage 10 of the present disclosure. The bandage 10 may comprise a strip 20 of material and a pad 30 for placement against the wound. The pad 30 may be centrally located, as shown, and comprise an absorbent material. The pad 30 may reside entirely within the strip 20, as illustrated. However, in some embodiments, one or more of the edges of the pad 30 may extend up to the edge of the strip 20.

As shown in FIG. 2, the strip 20 may include on its underside an adhesive 50 that is able to strongly bond to the skin of the patient when the bandage is applied to the patient, or to itself if the strip 20 is intended to wrap around. For example, the adhesive 50 may be of a pressure-sensitive type that allows secure adhesion to the patient's skin upon the application of force. The adhesive 50 should allow a bond sufficiently strong that the bandage 10 can stay securely in place as the patient moves about, and even in some cases when the bandage is exposed to water, moisture or humidity.

The ability of an adhesive to stick immediately is referred to as "tack." Tack is important in wound care dressings and other bandages because it is necessary to hold the dressing in the correct place. In an ideal scenario, there would be sufficient tack to secure the dressing in place during application, adhesion would keep the dressing in place for the duration of the appropriate wear time and the dressing would also permit removal without causing skin trauma, like tears or stripping.

Skin tears and stripping are always considerations when dealing with older and younger patients. The result of shearing, friction, or blunt trauma, skin tears result in the separation of the layers of skin. Skin tears are painful and can lead to infections and enlargement of the original wound. Accordingly, it is desirable to provide a bandage having adequate adhesion levels during device attachment, and low mechanical trauma during removal.

Accordingly, the adhesive 50 of the present disclosure can also allow easy peel-off of the bandage 10, with the goal of avoiding trauma, discomfort or pain to the patient. In addition to the factors and scenarios above, this is desirable where the bandage is placed on a sensitive region of the patient, such as on skin containing hair, skin lesions, cuts, scrapes, or other wounds. Likewise, certain types of wounds require frequent redressing with a fresh, clean bandage, while in other situations the duration of the application of the dressing is relatively short. Sometimes, the bandage is inadvertently positioned incorrectly. The ability to reposition the bandage without sacrificing its integrity (i.e., without loss of adhesion or deformation of bandage structure) and without causing trauma, discomfort or pain to the patient is desirable.

One exemplary adhesive suitable for achieving sufficient bond strength and also low trauma, peel-off releasability is a polyurethane mixture, which will be described in greater detail below. In certain embodiments, this polyurethane (PU) mixture comprises individual components of: (a) a polyol mixture, (b) an isocyanate, and (c) a catalyst mix. The PU mixture may be provided as a coating on the strip 20 of the bandage 10. To accomplish this, the individual components may be individually measured (weighed) before mixing. The PU adhesive mix may be poured in front of a graduated bar (e.g., wire of different thicknesses and spacing) which is drawn at a controlled speed over the strip, spreading the PU adhesive to a desired thickness. The strip can then be subjected to heat, such as by placing in an oven, to assist in the curing process.

The PU mixture may also be provided on a carrier substrate as a film which is then bonded to the strip 20. In this scenario, the same steps are performed as above, but the PU adhesive mix is placed on a carrier substrate. The substrate with adhesive serves as an adhesive film as opposed to an adhesive coating that can be bonded to the strip, or to any other surface requiring the adhesive properties described herein.

### EXAMPLES

### Example 1

### Thickness

Four different PU adhesives were produced having the following adhesive thicknesses which were generated from four different bars:

**Table 1. PU Adhesive Thicknesses**

| **Bar (µm)** | **Mean actual thickness (µm)** | **Range (µm)** |
|---|---|---|
| 8 (80) | 73 | 59-83 |
| 150 | 168 | 113-361 |
| 250 | 279 | 194-336 |
| 500 | 384 | 262-518 |

As reflected in Table 1 above, the mean thickness of the adhesive coating ranges from 73 to 384 µm.

### Example 2

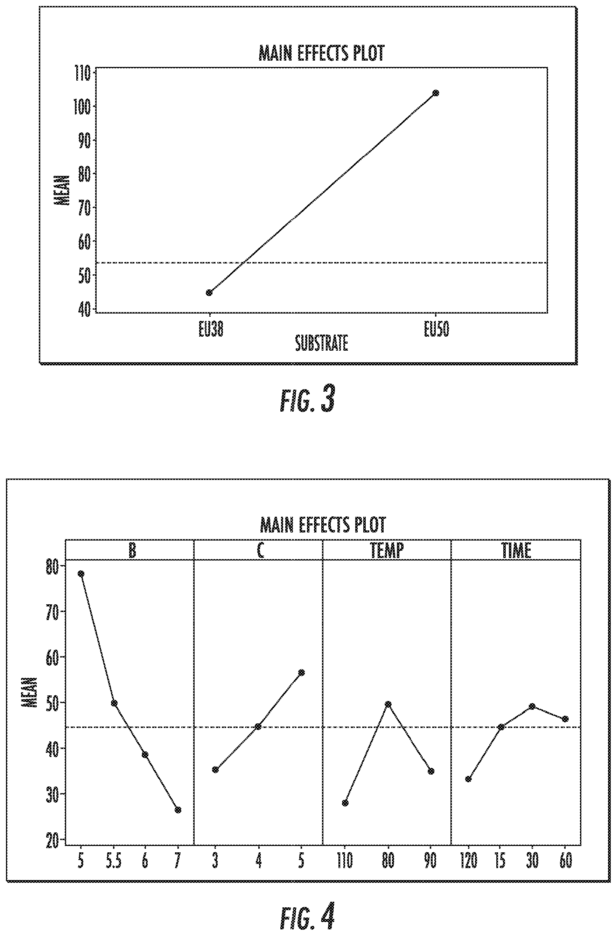

Two different substrate film types (EU38 and EU 50) with the PU adhesive coating of the present disclosure were produced and tested. An adhesion test using a metal plate was performed. The main effects plot results of the two examples of the PU adhesive coating with EU38 and EU50 film substrate, respectively, are plotted in the graph shown in FIG. 3. The EU50 film substrate had adhesion values over two times higher than the EU38 film. It is contemplated that the difference in the results of the adhesion test between film substrate EU38 and EU50 may be due to differences in higher moisture vapor transmission rate (mvtr) and water transmission rate (wtr), with the EU38 substrate film having higher values of moisture vapor and water transmission rates than the EU58 substrate film, and possibly accounting for a different chemistry and different results as shown.

### Example 4

FIG. 4 shows the main effects results of varying the catalyst content, isocyanate content, oven temperature and time in the oven for the EU38 film. As the graph indicates, adhesion decreases with increasing isocyanate content (graph labeled B), and increases with increasing catalyst (graph labeled C) In addition, adhesion decreases with increasing oven temperature (graph labeled TEMP). The exact impact of the time in the oven, however, is not yet clear (graph labeled TIME).

### Example 5

### Adhesion to Metal

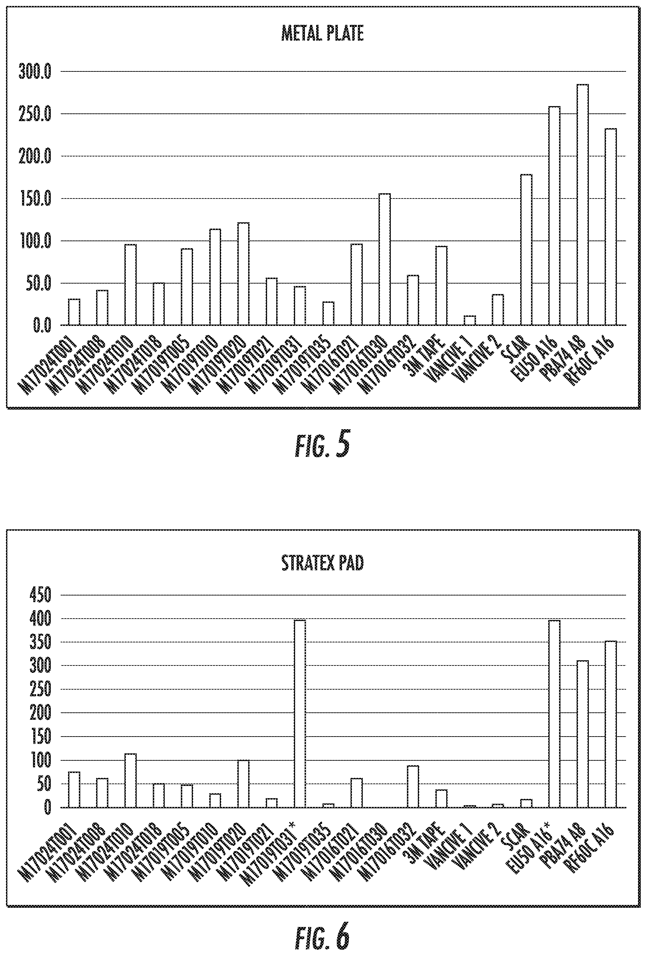

An exemplary PU adhesive according to the present disclosure (Stratex^{®} Gel Sheet 800-13) was produced and tested against other adhesives, including commercially-available acrylic adhesives and silicone adhesives, such as 3M^{®} and Vancive^{®}. Adhesives samples of about 50 mm wide were attached to a metal plate, which was then removed at an angle of 180° for the testing. The results of the adhesive comparison are shown in the bar graph of FIG. 5. As the title indicates, the adhesion test shows adhesion strength (g/cm) to a metal plate for the exemplary adhesive of the present disclosure (Stratex^{®} Gel Sheet 800-13), in comparison to other adhesives, such as silicone adhesives (e.g., 3M^{®} and Vancive^{®}), another PU adhesive (SCAR) and EU50 A16, RF60C A16 and PBA74 A8 adhesives. The trial samples of the present disclosure are labeled with an M (e.g., M17024T001, M17024T008, etc.). As shown, all of the trial samples except for one (M17016T030) produced adhesion values less than 150 g/cm to the metal plate. Many of the trial samples produced adhesion values of less than 100 g/cm (e.g., M17019T005) and some were below 50 g/cm (e.g., M17024T001 and M17024T008).

### Adhesion to Stratex^{®} Pad

The exemplary PU adhesive (Stratex^{®} Gel Sheet 800-13) was also tested for adhesion strength (g/cm) against other adhesives to a Stratex^{®} pad. The results are shown in the bar graph of FIG. 6. The majority of the samples tested on the metal plate were also adhered to the reverse side of the Stratex^{®} product 3.3NPET-E. In use as a first aid dressing, a pad could possibly be added and the ability of this pad to stick to the adhesive is important. Otherwise, a secondary adhesive operation may be required. Two samples, M17019T031 and the EU50A16, could not actually be removed from the pad, but have been given a value of 400 so they could be included on the chart of FIG. 6. Sample M17016T030 was not tested as no remaining sample available. It is noted that the silicone adhesive samples all had low adhesion to the Stratex^{®} pad.

Thus, the trial samples of the present disclosure generally had a higher adhesion to the pad than the commercially-available silicone adhesive samples, while still retaining a relatively low adhesion to the metal. This characteristic provides an optimized adhesive layer for a wound dressing, such as a bandage, because the adhesive will stick to the pad while still providing low trauma during removal from the patient.

### Adhesion to Embossed PE Film

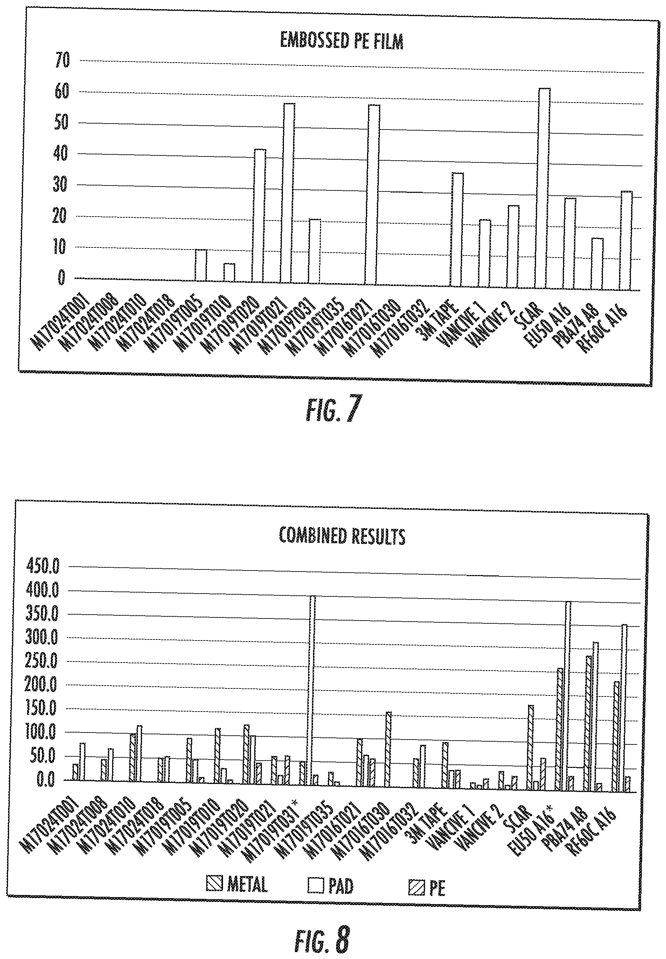

In addition, the exemplary PU adhesive (Stratex^{®} Gel Sheet 800-13) was tested for adhesion strength (g/cm) against other adhesives to an embossed polyethylene (PE) film. The results are shown in the bar graph of FIG. 7. It may be worth noting that, for this substrate, the acrylic adhesives which have shown clearly high adhesion levels to the metal and the pad also demonstrate high adhesion to the embossed PE film.

FIG. 8 represents a bar graph showing all of the adhesion data from FIGS. 5, 6 and 7 for the various adhesives tested, including the exemplary embodiment of PU adhesive described herein (Stratex^{®} Gel Sheet 800-13), in direct comparison with other adhesives for various different substrates.

### Example 6

### Water Immersion

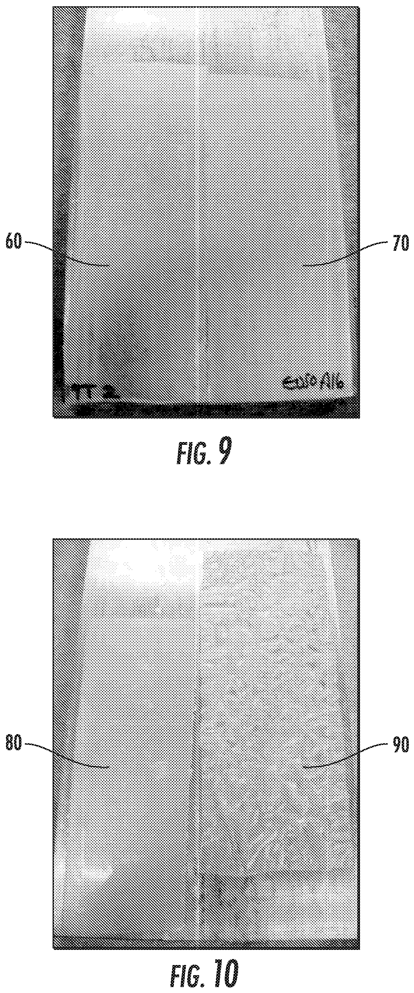

One of the critical parameters for an adhesive is that it can be used under wet conditions. Accordingly, testing was carried out wherein samples were stuck to a metal plate and placed under water. A visual assessment was made over periods of time from 1 to 30 minutes. FIG. 9 is a photograph showing a side-by-side comparison of the adhesive of the present disclosure and another adhesive, both after 30 minutes total immersion in water. As shown, the sample on the left is EU50 film substrate with the PU adhesive of the present disclosure (labeled 60 in FIG. 9), and the one on the right is EU50 film substrate with another adhesive, A16, both after 30 minutes total immersion in water (labeled 70 in FIG. 9). There appears to be some edge lift on the left with the PU adhesive, but greater than 95% of the adhesive remained adhered to the metal plate.

FIG. 10 is a photograph showing a side-by-side comparison of the adhesive of the present disclosure on two different film substrates, after 1 minute of total immersion in water. As shown, the sample on the left is film substrate CU684 (labeled 80 in FIG. 10) and the one on the right is substrate film EU38 (labeled 90 in FIG. 10), both with the PU adhesive of the present disclosure, after only 1 minute of total immersion in water. The sample made with EU38 substrate film is badly wrinkled and has started to fall off the plate. After a further 5 minutes, the sample is completely gone, while the CU684 sample is still mostly intact. This clearly demonstrates the influence of the film substrate on the behavior of the sample.

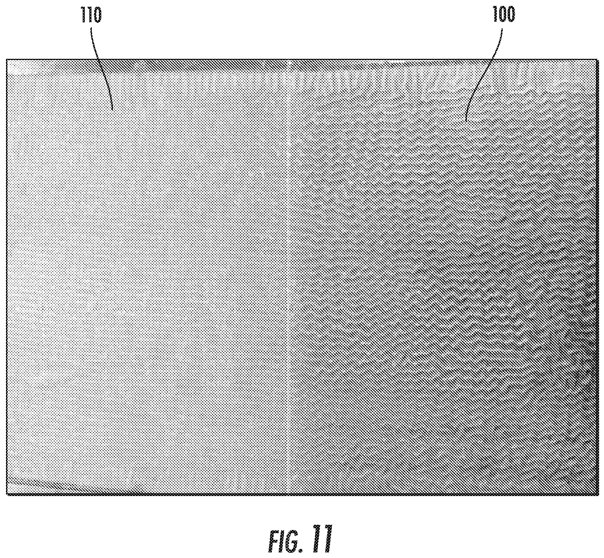

FIG. 11 is a photograph of the adhesive of the present disclosure on the EU38 substrate film immersed in water. Testing was conducted to assess whether the PU adhesive of the present disclosure would be impacted greatly by contact with water, since it is a polyurethane based gel. What was discovered from this test was that the type of substrate film onto which the PU adhesive was adhered had a strong influence on performance. Any samples which had the EU38 substrate film (having a relatively high moisture vapor transmission rate (mvtr) and water transmission rate (wtr) and labeled 100 in FIG. 11) wrinkled very quickly, and quickly led to the sample detaching from the plate. In contrast, those samples that had a film with a relatively low moisture vapor and water transmission rate (wtr), such as EU50 and CU684 substrate films (labeled 110 in FIG. 11), did not wrinkle, and the samples remained adhered to the plate for at least 30 minutes.

### Example 7

### Wrap Around

In the case of bandages 10 that are configured for use as a finger dressing, it is important that when the dressing is placed on the finger, it wraps around the finger and then is able to stick back onto itself. This was tested using the PU adhesive of the present disclosure as well as other adhesives. It was determined that all the trial PU film dressings stuck well to themselves, with the exception of some of the PBA74 samples. The 3M^{®} silicone tape and the Curad "Ouchless" silicone adhesive dressings demonstrated significantly less adhesiveness to themselves. Accordingly, the PU adhesives of the present invention provide an optimal finger bandage as they adhere to themselves significantly stronger than the conventional adhesives.

### Example 8

### Trauma on Removal

The ability to remove dressings from skin without the patient feeling pain is desirable for the adhesive 50 and bandage 10 of the present disclosure. Those patients with fragile skin, such as the elderly, babies or those that have suffered skin trauma do not need the added discomfort from a dressing that is painful to remove. Although no human testing was conducted or observed, it is believed that the adhesive of the present disclosure would allow a relatively easy release without much tugging of the skin to which the adhesive is attached.

Generally speaking, an exemplary reference guide is that an adhesion value of less than 100 is considered to be "low trauma", an adhesion value of between about 100 to 200 is considered medium trauma and an adhesive value of greater than 200 is considered to be high trauma, although there can be exceptions. It is important that the adhesive be cohesive. In other words, samples where there was adhesive left on the test plate often would test in the low trauma range, although they would not be considered low trauma because, in reality, they were not easy to peel off (having left residue behind). It is possible that in some tests, the adhesive testing actually gave a result for the adhesive failure.

Based on adhesion tests conducted, a wide range of adhesion values ranging from 20 to 120 g/cm were demonstrated for the samples of the present disclosure. As a reference, two samples of conventional acrylic adhesives had relatively high values of greater than about 200 g/cm for adhesion levels to the metal and the pad. In contrast, the PU adhesive samples resulted in lower adhesion values to the metal and the pad. The silicone adhesive samples were also lower trauma in that they produced lower adhesion values to the metal and the pad as well.

In most cases, the PU trial samples did not leave residue on the plate. Removal of the sample from the plate without leaving behind residue is an important consideration in determining whether there might be trauma to the patient, since any sample where adhesive is left behind on the plate would not be considered low trauma under these testing conditions.

From the adhesion testing to a metal plate, the acrylic adhesives are seen to have values above 200 g/cm and these can be classified as high trauma. One exemplary embodiment of the PU adhesive of the present disclosure has a value of >150 g/cm and can be considered medium trauma. Other exemplary embodiments of the present disclosure produced and tested had values of 100 g/cm or below and can be considered relatively low trauma.

It is recognized from these tests that the substrate on which the adhesives is applied affects performance. It is not desirable to have a substrate that is porous particularly if a liquid will be applied to it and it can seep through the substrate. The influence of a PU film on whether it can be coated at all, the adhesion level achieved and its behavior in contact with water have been observed through testing. The carrier type on which the film is stuck is also influential, not necessarily for the product properties, but for the ability to process the adhesive. Ideally, a relatively low moisture vapor transmission rate (mvtr)/ water transmission rate (wtr) film on a flexible PE/Pet/PP carrier is desirable.

### Example 9

### Repositionability

In most cases, for the trial PU adhesives, repositionability was possible even after 1 hour on the metal plate or the pad. Those samples wherein adhesive was left behind on the test plate or Stratex^{®} pad were treated as non-repositionable. In comparison, the acrylic adhesive samples were not as repositionable as the PU adhesives on immediate application or after any significant length of time. The 3M^{®} silicone tape and the Vancive^{®} silicone were repositionable with no adhesive residue. The "Ouchless^{™}" dressings were also repositionable with no adhesive residue.

In summary, the tests show that one can influence adhesion levels by varying materials and process of sample preparation. Using this sample preparation method, it is possible to consistently make an adhesive having a thickness of about 80 µm. When compared to acrylic adhesives, the exemplary PU adhesive appears to provide less skin trauma, and is comparable to the silicone adhesives tested. On the other hand, the PU adhesive appears to provide superior adhesion to the pad compared to the silicone adhesives tested. The selection of the substrate for the adhesive is critical to the ability to create an effective product and the performance of that product. When a highly breathable film was chosen, the film wrinkled quickly in water and caused the sample to fall off. With a lower mvp film, this did not occur and the film remained adhered to the plate.

In addition, samples made with the EU50 substrate film gave higher adhesion values than those made under the same conditions with the CU684 or EU38 substrate film. Further, the EU50 substrate film with PU adhesive did not wet out. The bandage 10 with PU adhesives 50 of the present disclosure allowed a finger dressing to wrap around and stick to itself. It is believed that, when PU adhesive samples are positioned on skin, it is possible to remove them immediately after application and replace them in the same or a different position without an apparent loss of adhesion. It is also believed that the PU adhesive samples can be repositioned after a longer period of time e.g., up to 1 hour. This is also possible with the silicone adhesives, but not possible with the acrylic adhesives unless they are repositioned almost immediately after the initial application. It is not possible to reposition the acrylic adhesive samples after any length of time.

Finally, although some of the PU samples did provide sufficient adherence to the pad, it is not yet determined what would be deemed an acceptable level of adherence. Certainly, those samples with low adhesion to the metal plate do have low adhesion to the pad. It seems likely that a PU adhesive can be produced that would stick to the pad.

It is contemplated that the adhesive and substrate carrying adhesive of the present disclosure may be adopted for many medical applications. While the illustrations show one exemplary embodiment of the adhesive in use with a bandage, the adhesive may be used with many other types of wound dressings. Additionally, the adhesive and substrate carrying adhesive can be used with medical fixation tape or medical patches, and can replace existing adhesives on many different medical devices including, for example, ostomy bags, medical tubes, and any wearable medical device having adhesive,

Other embodiments will be apparent to those skilled in the art from consideration of the specification and practice of the embodiment disclosed herein. It is intended that the specification and examples be considered as exemplary only, with a true scope of the embodiment being indicated by the following claims.

## Claims

1. A wound dressing, comprising:
a film base having a lower surface for contact with skin and an opposed, upper surface;
an absorbent pad component attached to the lower surface of the film base; and
an adhesive layer extending across a surface area of the lower surface of the film base,
wherein the adhesive layer comprises a polyurethane adhesive comprising a mixture of: (a) a polyol mixture, (b) an isocyanate, and (c) a catalyst mix and having a mean thickness in the range of about 73 to about 384 µm and an adhesion value of less than about 150 g/cm against a metal plate.

2. The wound dressing of claim 1, wherein the adhesive is a pressure-sensitive adhesive.

3. The wound dressing of claim 1, wherein the adhesion value is less than about 100 g/cm.

4. The wound dressing of claim 1, wherein the adhesive layer comprises a coating.

5. The wound dressing of claim 1, further comprising a flexible substrate film for carrying the adhesive layer.

6. The wound dressing of claim 5, wherein the flexible substrate film is bonded to the film base.

7. The wound dressing of claim 5, wherein the substrate film is selected from the group consisting of polyethylene, polyethylene terephthalate, polypropylene, polyurethane, ethylene vinyl acetate (EVA), polyester, copolyesters, and other polymer blends.

8. The wound dressing of claim 5, wherein the substrate film is a fabric, woven, non-woven, or sheet material.

9. The wound dressing of any one of the preceding claims, wherein the absorbent pad component is attached at a centrally located position on the lower surface of the film base.

## Patentansprüche

1. Wundverband, umfassend:
eine Folienbasis mit einer unteren Oberfläche für den Kontakt mit der Haut und einer gegenüberliegenden oberen Oberfläche, eine absorbierende Polsterkomponente, die an der unteren Oberfläche der Folienbasis angebracht ist; und eine Klebstoffschicht, die sich über einen Oberflächenbereich der unteren Oberfläche der Folienbasis erstreckt,
wobei die Klebstoffschicht einen Polyurethanklebstoff umfasst, der eine Mischung aus: (a) einer Polyolmischung, (b) einem Isocyanat und (c) einer Katalysatormischung umfasst, und die Klebstoffschicht eine mittlere Dicke im Bereich von etwa 73 bis etwa 384 µm und einen Haftwert von weniger als etwa 150 g/cm gegen eine Metallplatte aufweist.

2. Wundverband nach Anspruch 1, wobei der Klebstoff ein druckempfindlicher Klebstoff ist.

3. Wundverband nach Anspruch 1, wobei der Haftwert weniger als etwa 100 g/cm beträgt.

4. Wundverband nach Anspruch 1, wobei die Klebstoffschicht eine Beschichtung umfasst.

5. Wundverband nach Anspruch 1, ferner umfassend eine flexible Substratfolie zum Tragen der Klebstoffschicht.

6. Wundverband nach Anspruch 5, wobei die flexible Substratfolie an die Folienbasis gebunden ist.

7. Wundverband nach Anspruch 5, wobei die Substratfolie ausgewählt ist aus der Gruppe bestehend aus Polyethylen, Polyethylenterephthalat, Polypropylen, Polyurethan, Ethylenvinylacetat (EVA), Polyester, Copolyestern und anderen Polymermischungen.

8. Wundverband nach Anspruch 5, wobei die Substratfolie ein Stoff-, Gewebe-, Vlies- oder Blattmaterial ist.

9. Wundverband nach einem der vorstehenden Ansprüche, wobei die saugfähige Polsterkomponente an einer zentral angeordneten Position auf der unteren Oberfläche der Filmbasis angebracht ist.

## Revendications

1. Pansement pour plaie, comprenant :
une base en film ayant une surface inférieure destinée à être en contact avec la peau et une surface supérieure opposée ;
un composant de coussin absorbant fixé à la surface inférieure de la base en film ; et
une couche adhésive s'étendant sur toute la surface de la surface inférieure de la base en film,
dans lequel la couche adhésive comprend un adhésif polyuréthane comprenant un mélange de : (a) un mélange de polyols, (b) un isocyanate, et (c) un mélange catalytique, et ayant une épaisseur moyenne comprise entre environ 73 et environ 384 µm et une valeur d'adhérence inférieure à environ 150 g/cm sur une plaque métallique.

2. Pansement selon la revendication 1, dans lequel l'adhésif est un adhésif sensible à la pression.

3. Pansement selon la revendication 1, dans lequel la valeur d'adhérence est inférieure à environ 100 g/cm.

4. Pansement selon la revendication 1, dans lequel la couche adhésive comprend un revêtement.

5. Pansement selon la revendication 1, comprenant en outre un film substrat flexible destiné à supporter la couche adhésive.

6. Pansement selon la revendication 5, dans lequel le film substrat flexible est lié à la base du film.

7. Pansement selon la revendication 5, dans lequel le film de substrat est choisi parmi le groupe comprenant le polyéthylène, le polyéthylène téréphtalate, le polypropylène, le polyuréthane, l'éthylène-acétate de vinyle (EVA), le polyester, les copolyesters et d'autres mélanges de polymères.

8. Pansement selon la revendication 5, dans lequel le film de substrat est un tissu, un matériau tissé, non tissé ou en feuille.

9. Pansement pour plaie selon l'une quelconque des revendications précédentes, dans lequel le composant de coussin absorbant est fixé à une position centrale sur la surface inférieure de la base en film.
